(19) Europäisches Patentamt

European Patent Office

Office européen des brevets

(11) EP 1 177 808 A1

## (12) EUROPEAN PATENT APPLICATION

(43) Date of publication:
06.02.2002 Bulletin 2002/06

(51) Int Cl.⁷: **A61M 16/00**, A61M 16/20, F16K 17/00, F16K 1/00, F16K 27/00

(21) Application number: 01111002.0

(22) Date of filing: 07.05.2001

(84) Designated Contracting States:
AT BE CH CY DE DK ES FI FR GB GR IE IT LI LU
MC NL PT SE TR
Designated Extension States:
AL LT LV MK RO SI

(30) Priority: 01.08.2000 SE 0002805

(71) Applicant: **Siemens-Elema AB**
**171 95 Solna (SE)**

(72) Inventor: **Lundin, Bo**
**187 31 Täby (SE)**

(54) **Pressure relief valve**

(57) A fluid pressure relief valve (2) comprises an aperture (22) for the through-flow of a fluid, the aperture (22) being disposed between a fluid inlet 12 and a fluid outlet 24. A moveably mounted valving means (14,16,18) is disposed for pressure communication with the fluid and is moveable to unseal the aperture (22) when an opening force exerted by the communicated fluid pressure on the valving means (14,16,18) exceeds a counteracting threshold force. Biasing means (8) is adapted to provide a bias force for moving the valving means (14,16,18) towards sealing the aperture (22). The valving means (14,16,18) is disposed for pressure communication with the fluid such that a closing force less than and counter to the opening force is additionally exerted on the valving means (14,16,18) by the communicated fluid pressure, which force in combination with the bias force generates the threshold force.

Fig. 1

EP 1 177 808 A1

**Description**

The present invention relates to a pressure relief valve and in particular to a relief valve for regulating positive end expiratory pressure (PEEP) levels within a patient breathing circuit of a mechanical breathing assist device.

A pressure relief valve is a valve which exhibits a threshold resistance such that no fluid may flow through the valve until the fluid pressure equals a predetermined threshold value. At this time an opening force exerted by the fluid pressure on a valving element equals a counter-acting threshold closing force exerted by an actuator on the valving element. Above the threshold pressure it is important that the valve should allow high rates of fluid through-flow without any significant increase in pressure drop across the valve. This is because such a valve is typically employed to open in safety critical conditions where transfer of fluid pressure other than through the valve could have serious consequences. This is particularly true when the pressure relief valve is used as a safety valve within the patient breathing circuit. A pressure relief valve may also be employed within such a breathing circuit as a PEEP valve to control the positive end expiratory pressure and typically should be able to permit the through-flow of expiration gas at flow rates of up to 200 to 300 litres per minute without a significant increase in the pressure drop.

A pressure relief valve, such as a PEEP valve, typically comprises an aperture for the through-flow of a fluid towards which a moveably mounted valving means is urged with a predetermined threshold closing force generated by a biasing means. The valving means is a arranged with a sealing surface which co-operates with the aperture to regulate the through-flow of fluid and which can seal against the aperture with the predetermined closing force. The sealing surface is disposed for pressure communication with the fluid to be moveable in the direction of the fluid flow to unseal the aperture when an opening force exerted by the communicated fluid pressure exceeds the counter-acting closing force.

Since in the known prior art pressure relief valve all of the predetermined closing force is generated by the biasing means then relatively large and powerful means are required. Such a means is often space consuming and where the means comprises a solenoid, is often highly energy consuming too. Moreover, such biasing means are relatively expensive to produce.

It is known from US 6,082,705 to provide a flow regulation valve in which fluid, the flow of which is to be regulated, is used to seal the valve in the absence of an opening force provided by a biasing element. The valve comprises a valve housing having a fluid inlet and a fluid outlet between which is disposed a valve face. The valve face has a valve aperture adjacent to which is a moveable sealing element which is moveable by an opening force exerted by the fluid in the direction of fluid flow to unseal the aperture. A biasing means is provided to control the position of the sealing element and to regulate the flow of fluid through the valve. A duct is provided to divert a portion of the fluid to act on a side of the sealing element facing away from the aperture and so provide a closing force on the element counter to and at least as large as the opening force exerted by the fluid. In the absence of an opening force from the biasing means this will cause the element to move to seal the aperture.

It is an aim of the present invention to provide a pressure relief valve in which the use of a less powerful biasing element may be employed without reducing the threshold pressure of fluid required to open the valve.

According to the present invention there is provided a pressure relief valve as described in and characterised by the present Claim 1. By arranging for the valving means to be in pressure communication with the fluid such that both an opening force and a smaller closing force is generated on the valving means by the fluid pressure then the biasing element, which usefully may be adapted to generate a variable bias force, need only be powerful enough to contribute a bias force which is the difference between the closing force generated by the fluid pressure and a predetermined threshold force which must be matched by the opening force generated by the fluid pressure as it reaches a predetermined threshold pressure before fluid pressure is relieved by the valve.

Usefully, the valving element may comprise a first surface and a smaller second surface over which surfaces the fluid pressure acts and which are mechanically connected and disposed to define opposing end wall portions of a fluid receiving chamber having therein the aperture for the through-flow of the fluid which is sealable by the first surface when moved against the direction of through-flow of the fluid. This provides for a relatively simple valve construction. Moreover, by using the larger surface to seal the aperture then the aperture may be dimensioned to provide sufficient through-flow of fluid without a significant increase in the size of the valve.

Preferably, the valve is adapted to receive expiration gas from within an expiration line of a patient breathing circuit and to provide a PEEP level within the breathing circuit which may be made adjustable by providing a bias element capable of providing a variable bias force.

Exemplary embodiments of the present invention will now be described with reference to the drawings of the accompanying Figures, of which:

Fig. 1 shows a schematic illustration of an embodiment of the pressure relief valve according to the present invention.

Fig. 2 shows a schematic illustration of a further embodiment of the pressure relief valve according to the present invention.

Fig. 3 shows a schematic illustration of a patient breathing circuit according to the present invention.

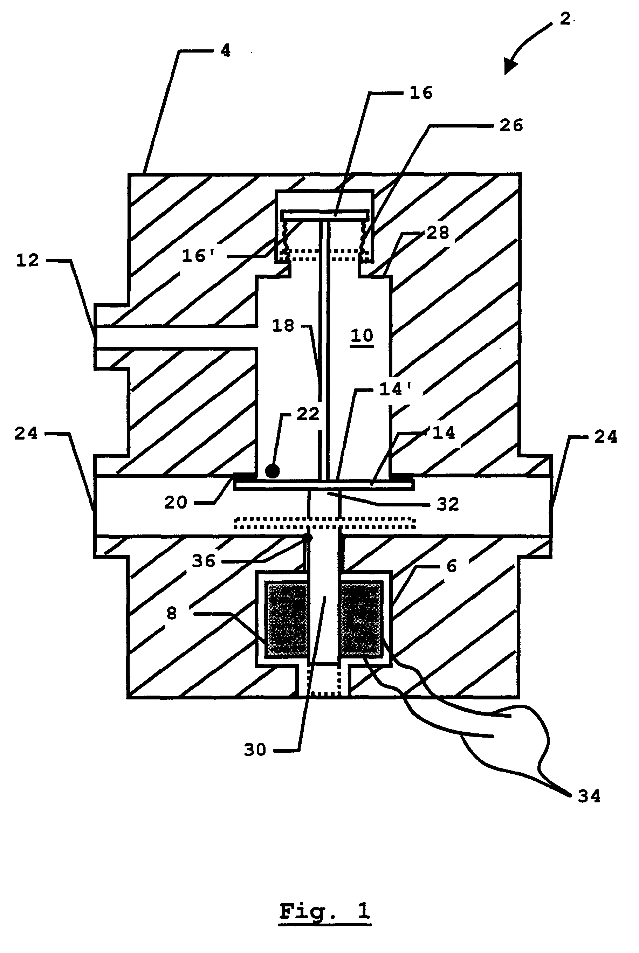

Consider now Fig. 1, an embodiment of a pressure relief valve 2 according to the present invention is illustrated partly in section. The valve 2 comprises a valve housing 4 having integral therewith a solenoid housing 6 in which is housed a solenoid 8. The valve housing 4 encloses a fluid receiving chamber 10 which connects to external the valve housing 4 via an inlet 12 through which fluid, such as an inspiration or an expiration gas, may be received into the chamber 10. The chamber 10 has opposing wall sections defined by moveable first 14 and second 16 plates which are mechanically connected by a rigid member 18 so that they are moveable in concert. The first plate 14 has a first surface 14' exposed to fluid pressure within the chamber 10 and the second plate 16 has a second surface 16' which is also exposed to the same fluid pressure. The area, A1, of the first surface 14' being greater than that, A2, of the second surface 16'. The first plate 14 is moveable to seal (solid lines in Fig. 1) and unseal (broken lines in Fig. 1) against a sealing ring 20 located about a periphery of an aperture 22 in the chamber 10. The aperture 22 connects to an outlet 24 through which fluid may leave the valve 2. The second plate 16 forms a rigid end wall of a collapsible wall section 26, the opposite end of which is sealed to a rigid wall section 28 of the chamber 10. The collapsible wall section 26 is arranged such that it is in an expanded state (as shown in Fig. 1) when the first plate 14 seals against the sealing ring 20 and is in a collapsed state when the plate 14 moves to unseal the aperture 22. A magnetically susceptible shaft 30 has an end 32 connected to the plate 14 and acts as a moveable core of the solenoid 8 such that by passing a current through the solenoid 8 via leads 34 the shaft 30 can urge the plate 14 towards the aperture 20 with a bias force, Fb, determined by the magnitude of the current flowing through the leads 34. Thus by varying the current through the leads 34 the bias force, Fb, can be varied. A sealing ring 36 is provided to prevent any leakage of fluid from the outlet 24 to the solenoid housing 6.

In use fluid at a pressure, P, enters the chamber 10 and produces a force, F1 on the first surface 14' of the first plate 14 having an area A1 over which the pressure, P, acts. The force, F1, is calculated to be:

$$F1 = P \cdot A1 \qquad (1)$$

which force, F1, acts on the first plate 14 to provide an opening force tending to unseal the aperture 22.

Simultaneously, the fluid pressure, P, acts on the smaller surface 16' of the second plate 16 which has an area A2, less than A1, to produce a force F2 on the plate 16. The force F2 is calculated to be:

$$F2 = P \cdot A2 \qquad (2)$$

which force, F2, because of the rigid connecting member 18, acts on the second plate 16 to provide a closing force on the first plate 14.

The closing force, F2, and the bias force, Fb, combine to generate a threshold force, Ft, which the opening force, F1, must reach before the plate 14 can move under the influence of a threshold fluid pressure to unseal the aperture 22 and relieve the fluid pressure. The threshold force Ft is then:

$$Ft = F2 + Fb = F1 \qquad (3)$$

Thus, if the valve of Fig.1 is used as a PEEP valve so that pressures above a selected PEEP pressure P(PEEP) cause the plate 14 to unseal the aperture 22 then the valve 2 must be devised such that from equations (1) to (3):

$$Fb = (A1-A2) \cdot P(PEEP) \qquad (4)$$

Since it is reasonable to expect the areas A1,A2 of the plates 14,16 to be known or readily obtainable and fixed then equation (4) may be used to calculate the necessary bias force, Fb, which the solenoid 8 must generate. This is less than the force which the solenoid 8 of prior art valves must generate since a part of the threshold force, Ft, in the valve 2 of the present invention is generated by the fluid pressure itself, acting on the smaller plate 16.

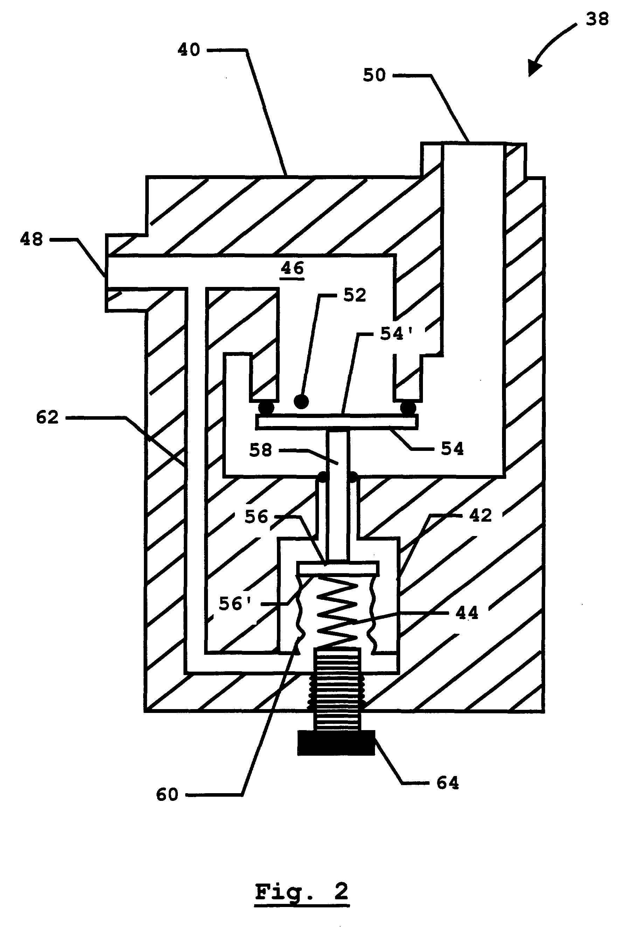

Considering now Fig. 2, a further embodiment of a pressure relief valve 38 according to the present invention is illustrated partly in section. The valve 38 comprises a valve housing 40 having integral therewith a spring housing 42 in which is housed a spring bias means 44. The valve housing 40 encloses a fluid duct which connects an externally accessible inlet 48 to an externally accessible outlet 50. An aperture 52 for the through flow of fluid is disposed within the duct 46 between the inlet 48 and the outlet 50 and is sealable by means of a first moveable plate 54 which is located directly in front of the outlet aperture 52 in the fluid flow direction through the valve 38, from inlet 48 to outlet 50 and is moveable to seal and unseal the aperture 54. The first plate 54 is mechanically connected to a second moveable plate 56 via a rigid rod 58 so that the two plates 54,56 can only move in concert. As with Fig.1 the first plate 54 is provided with a first surface 54' and the second plate 56 is provided with a smaller second surface 56' over which the fluid pressure may act. The second plate 56 is located within the spring housing 42 and is operably connected to the spring 44 such that a bias force, Fb', which is generated by the compression of the spring 44 is transmitted via the second plate 56 and the rod 58 to the first plate 54 to urge the plate 54 towards sealing the aperture 52. Also located within the spring housing 42 is a variable volume container 60 which is sealed at one end to the second plate 56 so as to vary its volume as the second plate 56 moves and encloses the spring 44. The interior of the container 60 is fluidly connected to the fluid duct 46 by means of a bypass duct 62. In this manner the second, smaller plate 56 is placed in pressure communication with fluid in the fluid duct 46, the pressure of which is to be regulated by the valve 38, only on one side of the plate 56.

The second surface 56' of the plate 56 has an area A2' and is urged upwards when exposed to a fluid at a pressure, P', with a closing force F2' as given by substitution into equation (2). The same fluid pressure within the fluid duct 46 generates an opening force, F1', on the first plate 54 whose first surface 54' has an area A1' at a level given by substitution into equation (1). If again the valve 38 is to act as a PEEP valve operating at a pressure P'(PEEP) then the bias force, Fb', necessarily provided by the spring 44 is, from equation (4):

$$Fb' = (A1'-A2') \cdot P'(PEEP) \qquad (5)$$

The required bias force, Fb', can be obtained by varying the compression of the spring 44 through rotating a threaded knob 64 attached to an end of the spring 44 either manually or automatically.

It will be appreciated by those skilled in the art that other known biasing means may be substituted for those 8,44 described in respect of Fig. 1 and Fig. 2 and that the described biasing means 8,44 can be substituted for one another without departing from the invention as claimed. Indeed, it may be preferable for safety reasons to substitute the solenoid 8 of the valve 2 of Fig.1 for a spring bias if the valve is used as a safety pressure release valve as a break in supply to the solenoid 8 may cause the valve to malfunction under safety critical conditions. Moreover, it will be further appreciated that the valve according to the present invention may simply modified without departing from the invention as claimed so that as an alternative the second, smaller, moveable plate 16, 56 is moveable to seal and unseal a through flow aperture.

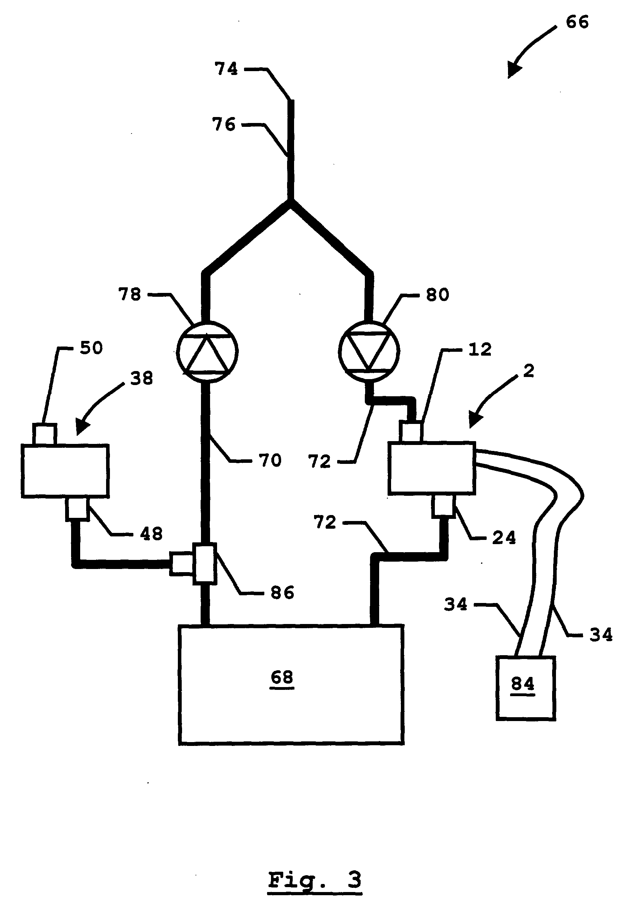

Consider now Fig. 3, an embodiment of a patient breathing circuit 66 according to the present invention is shown. The presently illustrated breathing circuit 66 represents a circuit as is generally known in the art which has included therein a known PEEP valve. An inventive difference of the breathing circuit 66 of Fig. 3 is that the known PEEP valve is substituted for a PEEP valve according to the present invention, for example a valve 2,38 according to the above described exemplary embodiments. For this reason the breathing circuit 66 shall not be described in great detail.

The breathing circuit 66 is shown to comprise a ventilator unit 68 to which ends of an inspiration gas line 70 and an expiration gas line 72 are connected for the transportation of gas respectively to and from an open end 74 of a patient tube 76, which open end 74 is intended in use to connect to the airways of a patient (not shown). One way valves 78, 80 are connected in-line in respective inspiration line 70 and expiration line 72 to ensure gas flows within the inspiration line 70 only in a direction towards the open end 74 and in the expiration line 72 only in a direction from the open end 74. A PEEP valve 2 according to the present invention is arranged in-line with the expiration line 72 with its inlet 12 connected to the expiration line 72 between the one way valve 80 and the ventilator 68 and its outlet 24 is connected via the expiration line 72 to the ventilator 68. The gas pressure within the expiration line 72 which will cause the valve 2 to open is controlled by current supplied by a control unit 84 (which may be incorporated within the ventilator 68) along leads 34 and is set so that the aperture 22 will remain unsealed until the pressure of the expiration gas falls to the PEEP level. A second relief valve 38 is incorporated in the inspiration line 70 and is arranged to act as a pressure release safety valve which operates to ensure that the pressure of inspiration gas supplied to the open end 74 does not exceed a predetermined safe maximum which may be set by adjusting the knob 64 (see Fig. 2) of the valve 38 to vary the extension of the spring bias 44 (see Fig. 2). To this end, and different to the PEEP valve 2 connection with the expiration line 72, only the inlet 48 of the valve 38 is connected (such as via a T-piece 86) to the inspiration line 70 with the outlet 50 venting to atmosphere (or a recovery system - not shown). In this configuration the aperture 52 will remain sealed until the inspiration gas pressure within the valve 38 exceeds a threshold set at the safe maximum value. Until this threshold is exceeded the inspiration gas continues to flow from the ventilator 68, along the inspiration line 70 and to the open end 74 of the patient tube 76.

It will be appreciated that the identical valves 2 or 38 may be used in the inspiration line 70 and the expiration line 72 of the breathing circuit 66 or that the valves 2 and 38 may be swapped to provide the functionality described above with respect to Fig. 3 without departing from the invention as claimed. Additionally or alternatively a valve 2, 38 according to the present invention may be included in-line in the inspiration line 70 and arranged in a manner similar to that shown for valve 2 in the expiration line 72 of Fig. 3 to act as a lower pressure delimiter for gas supplied from the ventilator unit 68.

**Claims**

1. A fluid pressure relief valve (2;38) comprising an aperture (22;52) for the through-flow of a fluid; a moveably mounted valving means (14,16,18;54,56,58) disposed for pressure communication with the fluid and moveable to unseal the aperture (22;52) when an opening force exerted by the communicated fluid pressure on the valving means (14,16,18;54,56,58) exceeds a counteracting threshold force; and biasing means (8;44) adapted to provide a bias force for moving the valving means (14,16,18;54,56,58) towards sealing the aperture (22;52) **characterised in that** the valving means (14,16,18;54,56,58) is disposed for pressure communication with the fluid such that a closing force less than and counter to the opening force is additionally exerted on the valving means (14,16,18;54,56,58) by the communicated fluid pressure, which force in combination with the bias force generates the threshold force.

2. A pressure relief valve as claimed in claim 1 **characterised in that** the valving means comprises a first surface (14';54') and a second surface (16':56') having respectively a first surface area and a smaller second surface area, the surfaces (14',16';54',56') being arranged such that the fluid pressure is communicated across the first surface (14';54') to generate the opening force and across the second surface (16';56') to generate the closing force.

3. A pressure relief valve as claimed in claim 2 **characterised in that** the valving means comprises a moveably mounted first element (14;54) having the first surface (14';54') and a spaced apart, mechanically connected, moveably mounted second element (16;56) having the second surface (16',56'), wherein the first element (14;54) is moveable to seal and unseal the aperture (22;52).

4. A pressure relief valve as claimed in claim 3 **characterised in that** the first (14) and the second (16) elements are disposed to define opposing end wall portions of a fluid receiving chamber (10); and **in that** the chamber (10) is provided with a fluid inlet (12) intermediate the opposing end wall portions (14;16).

5. A pressure relief valve as claimed in claim 3 or claim 4 **characterised in that** the first (14;54) and the second elements (16;56) comprise plates mechanically connected by a rigid connecting member (18;58).

6. A pressure relief valve as claimed in any of the previous claims **characterised in that** the valving means (14,16,18;54,56,58) is connectable in gas pressure communication with gas within a patient breathing circuit (66) and is co-operative with the biasing means (8;44) to seal the aperture (22;52) against through-flow of gas until the communicated gas pressure exceeds a desired pressure.

7. A pressure relief valve as claimed in claim 6 **char-**acterised in that** the valving means (14,16,18) is connectable with an expiration line (72) of the patient breathing circuit (66) and co-operative with the biasing means (8) to seal the aperture (22) against through-flow of expiration gas until the communicated gas pressure exceeds a desired positive end expiratory pressure (PEEP).

8. A pressure relief valve as claimed in any preceding claim **characterised in that** the biasing means (8;44) is operably connectable to control means (84;64) adapted to control the biasing means (8;44) to provide an adjustable bias force.

9. A patient breathing circuit (66) comprising an inspiration line (70) and an expiration line (72) one or both of which lines (70;72) having disposed therein a pressure relief valve **characterised in that** the pressure relief valve is a valve (2;38) as claimed in any preceding claim.

**Fig. 1**

**Fig. 2**

**Fig. 3**

<table>
<tr><td colspan="2">European Patent Office</td><td>EUROPEAN SEARCH REPORT</td><td>Application Number<br>EP 01 11 1002</td></tr>
</table>

## DOCUMENTS CONSIDERED TO BE RELEVANT

| Category | Citation of document with indication, where appropriate, of relevant passages | Relevant to claim | CLASSIFICATION OF THE APPLICATION (Int.Cl.7) |
|---|---|---|---|
| X,D | EP 0 911 054 A (SIEMENS ELEMA AB) 28 April 1999 (1999-04-28) | 1,2 | A61M16/00 A61M16/20 F16K17/00 F16K1/00 F16K27/00 |
| A | * the whole document * | 3-9 | |
| X | DD 273 982 A (MEDIZIN LABORTECHNIK VEB K) 6 December 1989 (1989-12-06) | 1,2 | |
| A | * the whole document * | 3-9 | |
| A | US 4 823 828 A (MCGINNIS GERALD E) 25 April 1989 (1989-04-25) * abstract; figure 1 * * column 2, line 37-50 * | 1-9 | |
| A | US 4 367 767 A (HURD CLAUDE C) 11 January 1983 (1983-01-11) * abstract; figures * | 1-9 | |

**TECHNICAL FIELDS SEARCHED (Int.Cl.7)**

A61M
F16K

The present search report has been drawn up for all claims

| Place of search | Date of completion of the search | Examiner |
|---|---|---|
| MUNICH | 13 November 2001 | Lager, J |

EPO FORM 1503 03.82 (P04C01)

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

**ANNEX TO THE EUROPEAN SEARCH REPORT
ON EUROPEAN PATENT APPLICATION NO.**

EP 01 11 1002

This annex lists the patent family members relating to the patent documents cited in the above–mentioned European search report.
The members are as contained in the European Patent Office EDP file on
The European Patent Office is in no way liable for these particulars which are merely given for the purpose of information.

13-11-2001

| Patent document cited in search report | | Publication date | Patent family member(s) | | Publication date |
|---|---|---|---|---|---|
| EP 0911054 | A | 28-04-1999 | EP | 0911054 A1 | 28-04-1999 |
| | | | JP | 11210927 A | 06-08-1999 |
| | | | US | 6098623 A | 08-08-2000 |
| DD 273982 | A | 06-12-1989 | DD | 273982 A1 | 06-12-1989 |
| US 4823828 | A | 25-04-1989 | NONE | | |
| US 4367767 | A | 11-01-1983 | NONE | | |

EPO FORM P0459

For more details about this annex : see Official Journal of the European Patent Office, No. 12/82